# EUROPEAN PATENT SPECIFICATION

(11) **EP 3 845 122 B1**
(45) Date of publication and mention of the grant of the patent: **29.07.2026**
(21) Application number: 19855295.2
(22) Date of filing: 25.04.2019
(51) Int. Cl.: A61B 5/02, A61B 5/00, G16H 40/67, G16H 50/30

(54) **MONITORING SYSTEM, AND METHOD AND DEVICE FOR DISPLAYING PHYSICAL SIGN PARAMETER**
ÜBERWACHUNGSSYSTEM UND VERFAHREN UND VORRICHTUNG ZUR ANZEIGE EINES KÖRPERLICHEN PARAMETERS
SYSTÈME DE SURVEILLANCE, ET PROCÉDÉ ET DISPOSITIF POUR L'AFFICHAGE DE PARAMÈTRE DE SIGNE PHYSIQUE

(30) Priority: 28.08.2018 CN 201810990861
(43) Date of publication of application: 07.07.2021
(73) Proprietor: Shenzhen Mindray Bio-Medical Electronics Co., Ltd., Shenzhen, Guangdong 518057 (CN)
(72) Inventor: TAN, Lin, Shenzhen, Guangdong 518057 (CN); YUAN, Weiwei, Shenzhen, Guangdong 518057 (CN); ZOU, Xiaoling, Shenzhen, Guangdong 518057 (CN)
(74) Representative: KIPA AB
(86) International application number: PCT/CN2019/084208
(87) International publication number: WO 2020/042639

(56) References cited:
- WO-A2-2013/056160
- CN-A- 101 272 734
- CN-A- 104 042 187
- CN-A- 109 171 755
- US-A1- 2009 005 703
- US-A1- 2011 071 420
- US-A1- 2015 138 205

## Description

This disclosure claims the priority of Chinese patent application No. 201810990861.7, filed with The State Intellectual Property Office on August 28, 2018 and entitled "MONITORING SYSTEM, AND METHOD AND APPARATUS FOR DISPLAYING PHYSIOLOGICAL PARAMETER".

### TECHNICAL FIELD

This disclosure relates to the field of medical technology, and more particularly to monitoring systems, and methods and apparatuses for displaying physiological parameter.

### BACKGROUND

Patient monitor can measure physiological parameters of a patient, and display measurement values of the physiological parameter, such that medical personnel can ascertain the basic condition of the patient by viewing the measurement values of various physical parameters. For example, the patient monitor measures parameter values of the patient's blood oxygen saturation, pulse rate and other physical parameters, and displays the measured values of the blood oxygen saturation, the pulse rate and other physical parameters, such that the medical personnel can ascertain the current state of the patient's blood oxygen saturation, the pulse rate and other physical parameters.

According to the description above, the medical personnel can only subjectively determine whether the current measurement value falls within the expected target range and whether it falls within the target range most of the time. It can be seen that the display mode of the current patient monitor is relatively simple, and the display of whether the current measurement value falls within the expected target range is not very intuitive, which cannot well meet the monitoring needs of the medical personnel. US2015/138205A1 defines a method for displaying physiological parameters.

### SUMMARY

In this regard, this disclosure provides a method for displaying physiological parameter, which is used to enrich the display content of a monitoring device to meet the monitoring needs of medical personnel.

In order to achieve the above purpose of the disclosure, this disclosure provides the following technical solutions.

In a first aspect, this disclosure provides a method for displaying physiological parameter, where the method includes: obtaining a measurement value of at least one physiological parameter of a monitored object, the measurement value of the physiological parameter being obtained from the monitored object by means of at least one sensor;
providing a monitoring interface, which comprises a state indication region that displays at least one graphical state indicator including multiple indication blocks, the multiple indication blocks respectively corresponding to multiple parameter value ranges of the physiological parameter, and the multiple indication blocks being displayed in the state indication region in an orderly arrangement according to a numerical value of the physiological parameter;
determining a parameter value range to which the measurement value of the physiological parameter belongs; and
performing display and output operations with respect to an indication block of the graphical state indicator corresponding to the parameter value range to which the measurement value belongs.

In a second aspect, this disclosure provides an apparatus for displaying physiological parameter, which includes:
a physiological parameter acquisition module for obtaining a measurement value of at least one physiological parameter of a monitored object, the measurement value of the physiological parameter being obtained from the monitored object by means of at least one sensor;
a display module for providing a monitoring interface, which comprises a state indication region that displays at least one graphical state indicator including multiple indication blocks, the multiple indication blocks respectively corresponding to multiple parameter value ranges of the physiological parameter, and the multiple indication blocks being displayed in the state indication region in an orderly arrangement according to a numerical value of the physiological parameter;
a parameter value range determination module for determining the parameter value range to which the measurement value of the physiological parameter belongs; and
a processing module for performing display and output operations with respect to an indication block of the graphical state indicator corresponding to the parameter value range to which the measurement value belongs.

In a third aspect, this disclosure provides a monitoring system, which includes:
a displayer, which is configured to display information; and
a processor, which executes a program instruction to implement the steps of the method for displaying the physiological parameter provided in the first aspect of this disclosure.

In a fourth aspect, this disclosure provides a readable storage medium with a computer program stored therein, wherein when the computer program is loaded and executed by a processor, the method for displaying the physiological parameter is implemented.

It can be seen from the above technical solutions that this disclosure provides a method for displaying physiological parameter. The method can provide, on a monitoring interface, a graphical state indicator for the physiological parameter, where the graphical state indicator includes multiple indication blocks, and the parameter value partitions corresponding to different indication blocks correspond to different physiological states. The method can also obtain the measurement value of the physiological parameter of the monitored object, determine the parameter value range to which the measurement value belongs, and indicate, in the graphical state indicator, the indication block corresponding to the parameter value range. In this way, the medical personnel can ascertain the current physiological state of the monitored object by means of viewing the indicated indication block.

### BRIEF DESCRIPTION OF THE DRAWINGS

FIG. 1 is a schematic flowchart of a method for displaying a physiological parameter;
FIG. 2 is a schematic diagram of a monitoring interface for blood oxygen saturation;
FIG. 3 is a schematic diagram of a monitoring interface for pulse rate;
FIG. 4 is a schematic diagram of a monitoring interface for perfusion index;
FIG. 5 is a schematic diagram of a monitoring interface for blood oxygen saturation, pulse rate and perfusion index;
FIGS. 6A-6F are multiple schematic diagrams of monitoring interfaces for blood oxygen saturation;
FIGS. 7A-7B are further schematic diagrams of monitoring interfaces for blood oxygen saturation, pulse rate and perfusion index;
FIG. 8 is a schematic structural diagram of an apparatus for displaying a physiological parameter; and
FIG. 9 is a structure diagram for a patient monitor.

### DESCRIPTION OF THE PREFERRED EMBODIMENTS

Current monitors can display measured value of some physiological parameters of a patient. However, the display mode is relatively simple, where only the numerical value of the measured value, or a trend chart and/or a waveform chart corresponding to the physiological parameter is displayed. The medical personnel can only subjectively determine whether the current measured value fall within an expected target range and whether it falls within the target range most of the time (a treatment target range is often not equal to an alarm range, and in the prior art, there was no explicit indication of whether the measured value is within the target range). The display of whether the current measured value falls within the expected target range is not very intuitive, and thus cannot intuitively provide corresponding diagnosis and treatment reference information for the medical personnel and cannot well meet the monitoring needs of the medical personnel.

This disclosure further provides a method for displaying a physiological parameter, which can improve the richness of the displayed parameter content and help the medical personnel to ascertain the current physiological state of the monitored object.

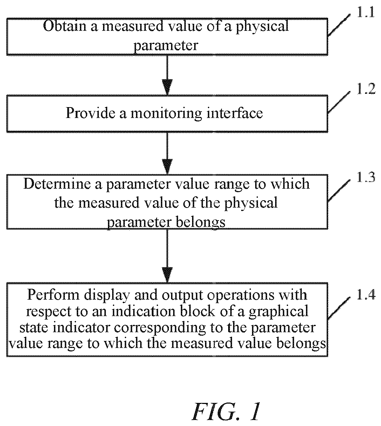

FIG. 1 shows a process of the method for displaying the physiological parameter.

As shown in FIG. 1, the process can specifically include steps 1.1-1.4.

At step 1.1: a measured value of a physiological parameter is obtained.

Specifically, a measured value of at least one physiological parameter of a monitored object is obtained, where the measured value of the physiological parameter is obtained from the monitored object by means of at least one sensor. The physiological parameter may be a parameter of any physiological sign of the monitored object, which for example can be heart rate, pulse rate, blood oxygen saturation, respiratory rate, etc.

The measured value of the physiological parameter collected by the sensor can be sent to a storage module of the monitoring system for storage, and these basic data can be used as basic data for display and analysis. That is to say, for the measured value of the physiological parameter, the measured value of the physiological parameter can be stored into the storage module of the monitoring system by means of the sensor, and then obtained from the storage module in this disclosure. Of course, the measured value of the physiological parameter may also be directly obtained from the sensor.

Therefore, the method for displaying the physiological parameter provided in this disclosure can be applied not only to bedside devices, but also to central stations. When applied to a bedside device, the measured value of the physiological parameter is obtained by means of various physiological parameter accessory devices; and when applied to a central station, the central station obtains the data of the physiological parameter from the bedside device via a network.

At step 1.2, a monitoring interface is provided.

Specifically, the monitoring interface includes a state indication region that displays at least one graphical state indicator including multiple indication blocks. Each of the indication blocks respectively corresponds to a parameter value range of the physiological parameter, and the multiple indication blocks are displayed in the state indication region in an orderly arrangement according to the numerical value of the physiological parameter.

The state indication region on the monitoring interface may be a region embedded in the monitoring interface, or may be a window region suspended on the monitoring interface. The position attribute, shape attribute, display attribute, state attribute, etc. of the state indication region can all be adjusted. For example, the position attribute of the state indication region refers to the display position of the state indication region on the monitoring interface; the shape attribute of the state indication region includes the pattern, the size, etc. of the shape (for example, the shape may be various shapes, such as rectangle, circle and heart shape); the display attribute of the state indication region refers to attribute information, such as color, brightness and contrast, of all or part of the region; and the state attribute of the state indication region includes attributes of being displayed or not displayed, being embedded in or suspended on the monitoring interface, etc.

The monitoring interface includes a chart containing multiple indication blocks, where different indication blocks correspond to different parameter value ranges of the physiological parameter, and different parameter value ranges can represent different physiological states of the monitored object. A visible chart has an intuitive indication of the physiological state, and for ease of description, the chart can be referred to as the graphical state indicator. The indication blocks are arranged in an orderly manner according to the numerical value of the physiological parameter, such as according to the order from left to right or from top to bottom, and the parameter value of the physiological parameter gradually increases along the orderly direction.

It should be noted that the number of the graphical state indicators is not limited to one, and may also be more than one. In the case where there are multiple graphical state indicators, different graphical state indicators correspond to different physiological parameters.

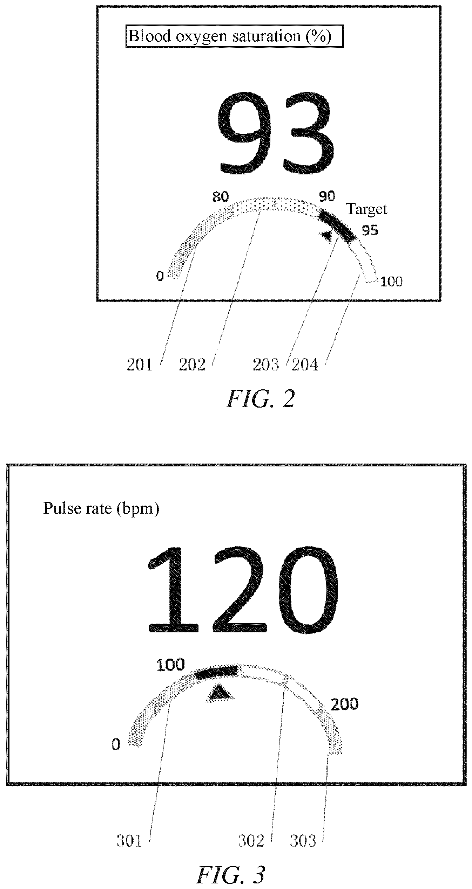

FIGS. 2-4 show the graphical state indicators respectively corresponding to blood oxygen saturation, pulse rate and perfusion index.

As shown in FIG. 2, the graphical state indicator for blood oxygen saturation (SpO2) includes four indication blocks. The indication block 201 corresponds to the parameter value range in which the blood oxygen saturation value is 0 to 80%, which is used to represent a high-severity state; the indication block 202 corresponds to the parameter value range in which the blood oxygen saturation value is 80%-90%, which is used to represent a moderate-severity state; the indication block 203 corresponds to the parameter value range in which the blood oxygen saturation value is 90%-95%, which is used to represent a treatment target state of blood oxygen saturation; and the indication block 204 corresponds to the parameter value range in which the blood oxygen saturation value is 95%-100%, which is used to represent a normal blood oxygen saturation state. Of course, four indication blocks are described above. In fact, each of the indication blocks can be further subdivided into multiple indication sub-blocks.

As shown in FIG. 3, the graphical state indicator for pulse rate (PR) includes three indication blocks. The indication block 301 corresponds to the parameter value range in which the pulse rate value is 0-100, which can represent a low pulse rate state; the indication block 302 corresponds to the parameter value range in which the pulse rate value is 100-200, which can represent a physiological alarm range of pulse rate; and the indication block 303 corresponds to the parameter value range in which the pulse rate value is greater than 200, which can represent a state where the pulse rate is too fast. The unit of pulse rate value is beat per minute (bpm). Of course, three indication blocks are described above. In fact, each of the indication blocks can be further subdivided into multiple indication sub-blocks.

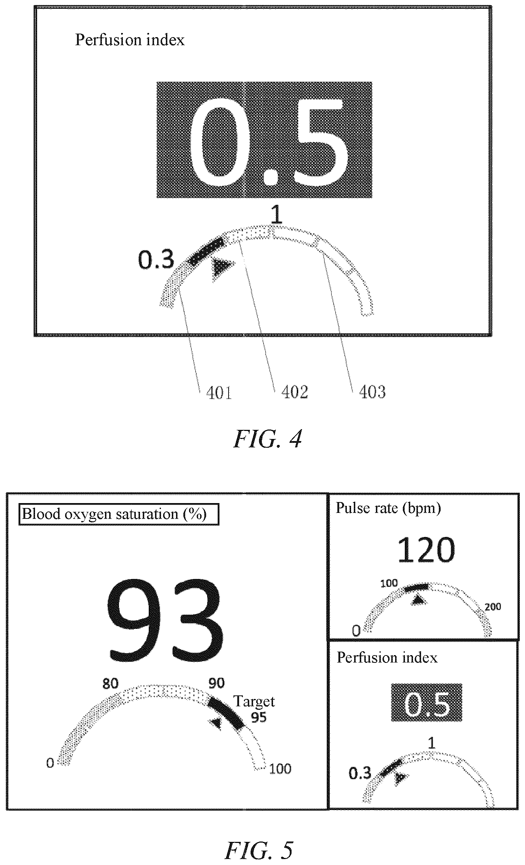

As shown in FIG. 4, the graphical state indicator for perfusion index (PI) includes three indication blocks. The indication block 401 corresponds to the parameter value range in which the perfusion index value is 0-0.3, which can represent the state where the patient's weak perfusion is severe, and since the weak perfusion is too low, the measured blood oxygen saturation value may not be accurate enough at this time; the indication block 402 corresponds to the parameter value range in which the perfusion index value is 0.3-1, which can represent that the patient has a weak perfusion problem currently; and the indication block 403 corresponds to the parameter value range in which the perfusion index value is greater than 1, which can represent that the patient has good perfusion condition currently. Of course, three indication blocks are described above. In fact, each of the indication blocks can be further subdivided into multiple indication sub-blocks.

It should be noted that for different physiological parameters, the parameter value ranges corresponding to the indication blocks are different, and can be set according to the clinical guidance significance. In addition, in order to help the medical personnel to ascertain the range of parameter-value partitions in the graphical state indicator, as shown in FIGS. 2-4, the graphical state indicator includes critical values of the parameter-value partition.

In the case where there are multiple physiological parameters, the graphical state indicators of the multiple physiological parameters can be simultaneously displayed on the monitoring interface. As shown in FIG. 5, the monitoring interface includes graphical state indicators for three physiological parameters, including blood oxygen saturation (SpO2), pulse rate (PR) and perfusion index (PI).

At step 1.3, the parameter value range to which the measured value of the physiological parameter belongs is determined.

Specifically, monitoring data that comprises the measured value of the physiological parameter can be generated by monitoring the target object. The graphical state indicator of the physiological parameter includes multiple parameter value ranges, and according to the numerical value of the measured value of the physiological parameter, the parameter value range to which the measured value belongs is determined. The measured value of the physiological parameter may specifically be the currently measured parameter value, i.e., the real-time measured value.

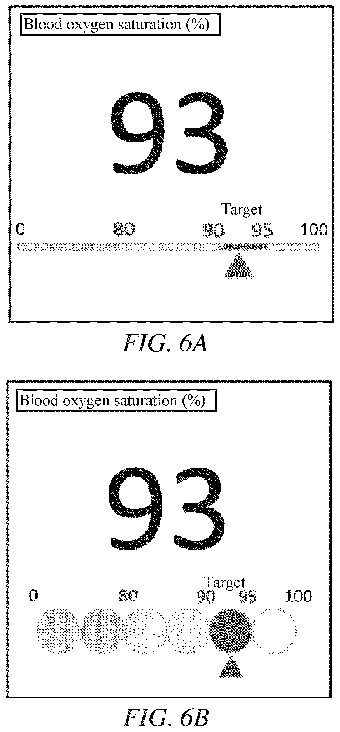

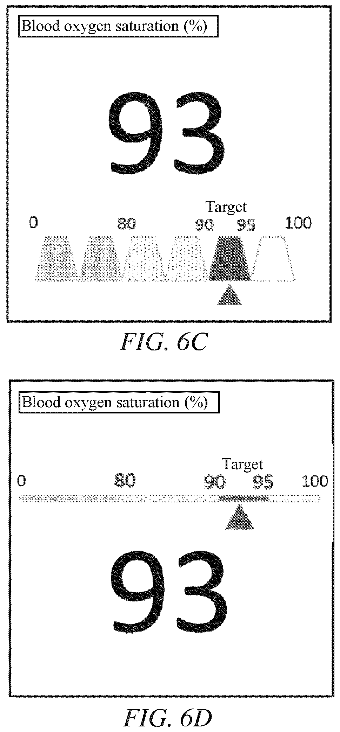

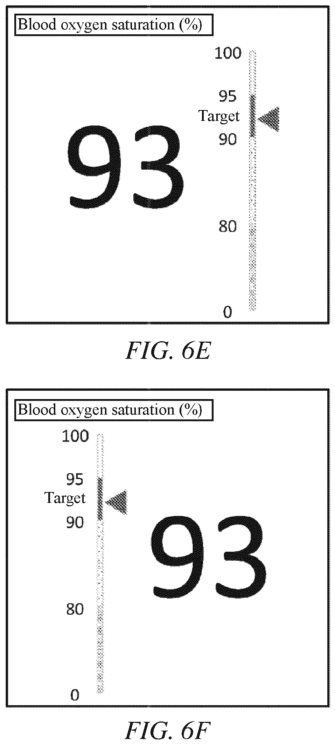

For example, the physiological parameter is blood oxygen saturation, and as shown in FIG. 2, the graphical state indicator for blood oxygen saturation includes four parameter value ranges. Assuming that the measured value of the blood oxygen saturation of certain monitored object is 93%, it can be seen from the four parameter value ranges divided in FIG. 6 that the parameter value range to which the measured value belongs is the parameter value range corresponding to 90%-95%.

At step 1.4, display and output operations is performed in the indication block of the graphical state indicator, which indication block corresponds to the parameter value range to which the measured value belongs.

The graphical state indicator includes multiple indication blocks, and different indication blocks correspond to different parameter value ranges. After the parameter value range to which the measured value belongs is determined, the indication block corresponding to the parameter value range is indicated. Different display modes are used to differentiate the display of the multiple indication blocks of the graphical state indicator. The display mode may include differentiating by color, highlighting, adding indication marks, etc., and the specific indication mode will be described in detail below in conjunction with the accompanying drawings, which will not be repeated here. Alternatively, the display mode may also be other modes, as long as it can be differentiated from other parameter-value partitions.

Different parameter value ranges represent different physiological states, and the indication about which parameter value range the measured value belongs to can indicate the medical personnel what physiological state the monitored object has at the time point when the measured value is collected. Still taking FIG. 2 as an example, in the graphical state indicator, the range of the parameter value range of 90%-95% represents the treatment target state of the blood oxygen saturation, and if the parameter value range to which the measured value belongs is this target range, it represents that the physiological state of the monitored object has reached the desired target state of the treatment.

It can be seen from the above technical solutions that this disclosure provides a method for displaying the physiological parameter. The method can provide, on the monitoring interface, the graphical state indicator for the physiological parameter, where the graphical state indicator includes multiple indication blocks, and the parameter-value partitions corresponding to different indication blocks correspond to different physiological states; the method can also obtain a measured value of the physiological parameter of the monitored object, determine the parameter value range to which the measured value belongs, and indicate, in the graphical state indicator, the indication block corresponding to the parameter value range. In this way, the medical personnel can ascertain the current physiological state of the monitored object by means of viewing the indicated indication block.

With the development of detailed monitoring, the evaluation of the measured value in clinical practice is no longer limited to a single state of whether the measured value exceeds an alarm threshold. Instead, the medical personnel hope to be able to perform more patient status grading for the measured value. The graphical state indicator provided in this disclosure includes multiple parameter value ranges, different parameter value ranges correspond to different physiological states, and the medical personnel can perform detailed monitoring for a variety of different physiological states of the monitored object by means of viewing the graphical state indicator.

It will specifically illustrate how to indicate, in the graphical state indicator, the indication block corresponding to the measured value.

In an embodiment, each of the multiple indication blocks of the graphical state indicator is provided with an initial brightness value and a preset brightness value. The initial brightness value refers to a brightness value which represents that the measured value does not correspond to the indication block; and the preset brightness value refers to a brightness value which represents that the measured value corresponds to the indication block of the brightness value.

Therefore, in order to indicate the indication block corresponding to the measured value, the display brightness of the indication block corresponding to the parameter value range to which the measured value belongs can be increased from the initial brightness value to the preset brightness value in the graphical state indicator. In order to achieve better indication effect, the initial brightness value is low brightness, and the preset brightness value is high brightness. That is, the indication block corresponding to the parameter value range to which the measured value belongs is highlighted.

The multiple indication blocks may have the same initial brightness values and the same preset brightness values. After the indication block corresponding to the measured value is determined, only the indication block corresponding to the measured value is displayed with brightness adjustment. Alternatively, the multiple indication blocks may also have different initial brightness values and different preset brightness values.

In an embodiment, each of the multiple indication blocks of the graphical state indicator is provided with an initial color and a preset color. The initial color represents that the measured value does not correspond to the indication block of the initial color value; and the preset color value represents that the measured value corresponds to the indication block of the preset color value.

Therefore, in order to indicate the indication block corresponding to the measured value, the display color of the indication block corresponding to the parameter value range to which the measured value belongs can be increased from the initial color to the preset color in the graphical state indicator. In order to achieve better indication effect, the initial color is light, and the preset color is dark. That is, the indication block corresponding to the parameter value range to which the measured value belongs is displayed in a darker color.

The multiple indication blocks may have the same initial color and the same preset color. After the indication block corresponding to the measured value is determined, only the indication block corresponding to the measured value is displayed in a darker color. Alternatively, the multiple indication blocks may also have different initial colors and different preset colors.

In some embodiments, it may be a combination of the above two embodiments.

In an embodiment, the multiple indication blocks of the graphical state indicator include a target indication block used to represent the expected state of the physiological parameter. Therefore, if the parameter value range to which the measured value belongs is the parameter value range corresponding to the target indication block, the target indication block is displayed in a display and output mode differentiated from other indication blocks.

The expected state of the physiological parameter may include any one of the states such as a normal state, an abnormal state, and a treatment target state. The target indication block refers to the indication block representing a certain expected state in the multiple indication blocks. If the measured value corresponds to the target indication block, the target indication block is displayed and outputted differently from other indication blocks.

There may be many modes for differentiating the indication blocks. For example, if the display and output mode of the indication block is to adjust the display brightness, the mode for differentiating the indication blocks may refer to adjusting the display brightness value for the target indication block to be larger or smaller. For another example, if the display and output mode of the indication block is to switch the display color, the mode for differentiating the indication blocks may refer to that the display color of the target indication block is different from that of other indication blocks after display color switching. For another example, the target indication block is elliptical in shape, and the non-target indication block is rectangular in shape. For another example, the target indication block is displayed with shading filling, and the non-target indication block is not displayed with shading filling. As another example, it may be a combination of the above modes or other differentiating modes.

The display and output mode will be illustrated below taking an example in which the expected state is a treatment target state.

The treatment target state represents the state that the monitored object needs to reach after the treatment means in a monitoring scenario. This state may be an expected treatment state generally recognized in the medical field for all monitored objects, or may also be an expected treatment state that is set for a specific monitored object and that the specific monitored object needs to reach in a specific monitoring scenario.

The parameter value range corresponding to the treatment target state may be referred to as the parameter value range of the treatment target value, that is to say, the parameter values belonging to the parameter value range are all the treatment target values. Also, in the multiple indication blocks, the indication block corresponding to the parameter value range of the treatment target value may be referred to as the treatment target indication block.

In order to make a prompt for the treatment target indication block, a prompt will be added in the graphical state indicator at the corresponding position of the treatment target indication block. The prompt may be in text form, such as "target" in Chinese (or "target" in English) in FIGS. 2 and 5 above and in FIGS. 6A-6F below. Alternatively, the prompt may also be a picture or a symbol, such as a picture or a symbol of a smiley face. Alternatively, the prompt may also be in other forms.

When the measured value of the physiological parameter is determined to jump from a parameter value range to which the non-treatment target value belongs to a parameter value range to which the treatment target value belongs, the display and output operations are performed with respect to the treatment target indication block in a preset display pattern differentiated from the original display pattern.

The original display pattern represents that the measured value does not correspond to the treatment target indication block; and the preset display pattern represents that the measured value corresponds to the treatment target indication block. The measured value jumps from a parameter value range to which the non-treatment target value belongs to a parameter value range to which the treatment target value belongs, which represents that the measured value jumps from a state that does not correspond to the treatment target indication block to a state that corresponds to the treatment target indication block, and thus the display pattern needs to jump from the original display pattern to the preset display pattern.

Of course, if the measured value jumps to the opposite situation, i.e., jumps from the parameter value range to which the treatment target value belongs to the parameter value range to which the non-treatment target value belongs, the display pattern can jump reversely, i.e., canceling the preset display pattern, and performing display and output operations with respect to the treatment target indication block in the original display pattern.

The display pattern may include brightness value and/or display color. The specific jump mode can be illustrated with reference to the first two embodiments in this part.

In an embodiment, in order to indicate the indication block corresponding to the parameter value range to which the measured value belongs, an indication icon may be displayed at the position of the indication block corresponding to the parameter value range to which the measured value belongs. The indication icon points to the indication block corresponding to the parameter value range to which the measured value belongs.

The indication icon may be a cursor pointer, such as the triangular cursor pointer in FIGS. 2-6F. The position where the indication icon is added needs to be able to prompt the medical personnel, i.e., to indicate the medical personnel which indication block the measured value corresponds to. For example, when the graphical state indicator is a horizontally placed straight-bar structure, the indication icon is added directly above or below the indication block. For another example, when the graphical state indicator has an arc structure, the indication icon is added at the central position of the arc of the indication block, etc.

It should be noted that the several embodiments mentioned above can be combined arbitrarily. For example, after the indication block corresponding to the measured value is determined, the preset color of this indication block is highlighted, and an indication icon is added at the corresponding position of the indication block.

Further, in order to enable the monitoring personnel to ascertain the physiological state of the monitored object, the measured value can be displayed in the state indication region of the monitoring interface, and/or the waveform chart and/or the trend chart corresponding to the physiological parameter can be displayed in the state indication region of the monitoring interface. For example, for the measurement of the blood oxygen saturation, a tracing wave (a waveform chart) with respect to the blood oxygen saturation or a blood oxygen saturation trend chart (the chart depicted based on the blood oxygen saturation values obtained by continuous measurement over a period of time) can be displayed in the state indication region.

The font and size of the measured value can be preset, or can be modified. To enable the prompt obviously, the font may be a relatively large-size font. That is, a large-font display mode is used.

The display position of the measured value may be freely arranged, or may be set according to the position of the graphical state indicator. The graphical state indicator and the measured value may be in the relative position of up and down, left and right, etc. The graphical state indicator may be located above the measured value, or the two have opposite positions; and the graphical state indicator may be located on the left of the measured value, or the two have opposite positions.

Referring to the monitoring interface shown in FIGS. 6D-6F below, the current measured value of the blood oxygen saturation of 93% is respectively located below, at the left, and at the right of the graphical state indicator.

The physiological parameter may have an alarm threshold. If the measured value exceeds the alarm threshold, the measured value can be displayed according to the preset alarm prompt mode to prompt the medical personnel that the target physiological parameter of the monitored object is abnormal. The preset alarm prompt mode may include any one or more of: displaying in an inverse color, changing from a non-alarm color to an alarm color such as red, adding shading background, and flashing. As shown in FIGS. 4 and 5, the measured value of the perfusion index of 0.5 exceeds the alarm threshold, and thus the numerical value is inverted from black to white, and a gray shading background is added. Of course, the alarm prompt mode may also be other modes and is not limited thereto. It should be noted that the alarm threshold may include multiple alarm thresholds. Different alarm thresholds correspond to different inverse colors, and if an alarm threshold is exceeded, the color needs to be inverted to the corresponding color. For example, the inverse color of high-level alarm threshold is red, and the inverse color of intermediate-level alarm threshold is yellow.

The graphical state indicator is formed by connecting multiple indication blocks. Regardless of whether the indication blocks are of the same shape or different shapes and whether the shape is regular or irregular, the multiple indication blocks are connected in such a manner that forms a connection line, and the shape of the connection line will determine the overall shape of the graphical state indicator. For example, for the graphical state indicator in FIGS. 3-5, the connection lines of the multiple indication blocks are arc-shaped lines, and thus the overall structure of the formed graphical state indicator is arc-shaped.

Alternatively, the graphical state indicator is not limited to be arc-shaped, but may also be in another shape. FIGS. 6A-6C are other implementations of the graphical state indicator shown in FIG. 2. As shown in FIG. 6A, the connection line of each indication block in the graphical state indicator is in a straight-line shape, so it can be considered that the structure of this graphical state indicator is in a straight-bar or straight-line shape. It should be noted that the graphical state indicator can be placed horizontally, vertically or at an angle in the case of the straight-line shape.

It should be noted that, no matter of what connection shape of the graphical state indicator, the indication block may be either in a long-bar shape or a line shape. In this case, the graphical status indicator having the arc-shaped structure is similar to an instrument panel. In addition, as shown in FIGS. 6B and 6C, the indication blocks in the graphical state indicator may also be respectively circular or trapezoidal in shape. Alternatively, the indication block may also be in other shapes, and is not limited to what is shown in the accompanying drawings.

It can be seen from the above drawings that the graphical state indicators of any form all include multiple indication blocks, and different indication blocks correspond to different parameter-value partitions of the physiological parameter. The change of the parameter value can reflect the state change of the monitored object in this physiological parameter. Quantitative changes will cause qualitative changes. A parameter value in a certain range may represent a state of the monitored object, such as a normal state, and a parameter value in another range may represent another state of the monitored object, such as an abnormal state. Therefore, parameter-value partitions of a physiological indicator can be set according to the clinical experience, and different parameter-value partitions correspond to different physiological states.

It can be understood that when there are more parameter-value partitions, more physiological states can be reflected, and thus the monitoring of the monitored object can become more detailed. For example, there may be two parameter-value partitions, respectively representing the normal state and the abnormal state. For another example, there may be three parameter-value partitions, respectively representing the normal state, the mildly abnormal state, and the severely abnormal state.

**In** the embodiment described above, the measured value of the physiological parameter is displayed in the state indication region. In some embodiments, the monitoring interface may also include the measurement parameter display region, and the measurement parameter display region and the state indication region are two adjacent regions with the common boundary. The measured value is displayed in the measurement parameter display region.

Specifically, the measurement parameter display region and the state indication region are two independent regions, and the two regions are positioned adjacently. The adjacent relationship between the state indication region and the measurement parameter display region does not include a closed surrounding relationship, but may include a semi-closed encompassing relationship. More specifically, the measurement parameter display region may be located beyond the arc of the state indication region, such as directly above or at the upper left of the state indication region, that is to say, the state indication region does not surround the measurement parameter display region.

In addition, the boundary lines of the measurement parameter display region and the state indication region may be displayed together or hidden together, or one of them is displayed while the other is hidden.

The multiple indication blocks of the graphical state indicator are arranged in a straight-bar shape or a circular arc shape in an orderly manner, and a parameter pointer is displayed in the state indication region with one end thereof indicating the position of the indication block corresponding to the parameter value range to which the measured value belongs. It can also be seen from the circular arc shape that the graphical state indicator is not a closed circle, which further indicates that the graphical state indicator does not completely surround the measured value. The description regarding the parameter pointer can refer to the description of the indication icon mentioned above, which will not be repeated here.

When multiple indication blocks of the graphical state indicator are arranged in a circular arc shape in an orderly manner, one end of the parameter pointer can indicate the position of the indication block corresponding to the parameter value range to which the measured value belongs, and the other end thereof can be displayed at the center position corresponding to the circular arc shape. Such a display effect is similar to the instrument panel, where the measured value is the value in the instrument panel, one end of the pointer is at the center position of the instrument panel, and the other end of the pointer points to the measured value in the instrument panel. It should be noted that because the state indication region does not surround the measurement parameter display region, the parameter pointer will have such a display pattern.

The multiple indication blocks of the graphical state indicator are arranged in a circular arc shape in an orderly manner. In some embodiments, the circular arc shape may be in the shape smaller than a semicircle, i.e., the central angle corresponding to the circular arc shape is less than 180 degrees. In this way, the height of the graphical state indicator can be limited, such that the measured value can be displayed in a large-font display pattern to highlight the measured value.

The large-font display pattern of the measured value refers to the pattern that can affect the size of the region occupied by the measured value, such as font, font size, and font bold. That is to say, in order to achieve the large-font display pattern, a preset large font, a preset large font size and font bold can be used for the measured value. Through the large-font display pattern, the measured value can be noticeably displayed to have a reminding effect for the measured value.

In addition to viewing the measured physiological state of the monitored object, it is also needed to ascertain the historical physiological state of the monitored object. The combination thereof can provide the medical personnel with a richer diagnosis basis. Therefore, the historical values of physiological parameter can be analyzed, and the analysis result is displayed on the monitoring interface.

In an analysis mode, distribution statistics can be performed on the historical values. Specifically, the historical values of at least one physiological parameter of the monitored object within a preset time duration are obtained; distribution statistics are performed on the historical values based on at least one parameter-value partition, and a distribution statistic result corresponding to the parameter-value partition is determined; and a segment statistic region is provided on the monitoring interface, and the distribution statistic result is displayed in the segment statistic region.

It can be understood that there are a large number of historical values of the physiological parameter, and not all the historical values have guidance significance for the current diagnosis. According to the actual monitoring needs, the time duration can be preset to limit which time points within which the historical values need to be obtained. For ease of description, the set time duration can be referred to as the preset time duration.

It should be noted that the preset time duration is not limited in the form, and may be either a continuous historical time period or multiple discontinuous historical time points. When the preset time duration is a continuous time period, the end time point may be either the current time point, or a time point before the current time point. When the preset time duration are multiple historical time points, the time points may have either the same time duration of interval or different time durations of interval therebetween. It can be seen that the preset time duration may not only refer to a duration within a time window, but may also include the location of the time window, that is, limiting the start and end time locations of the time window.

After the historical values of the physiological parameter are obtained, statistics analysis can be performed on the historical values according to the preset parameter-value partition. It should be noted that the parameter-value partition may be a segment in the time, or a segment in the numerical value range of the historical value, or a combination thereof, or other partition conditions. It should be noted that the parameter-value partition here may be different from the parameter value range corresponding to the indication block in the above graphical state indicator.

If there are multiple parameter-value partitions, after the distribution statistics, the respective distribution statistic result can be obtained corresponding to each parameter-value partition. Alternatively, there may also be one parameter-value partition, which refers to a specific case where all the historical values are taken as one distribution statistic result.

The segment statistic region is provided on the monitoring interface for displaying the distribution statistic result. There may be one or more physiological parameters. When there are multiple physiological parameters, the segment statistic region can be divided into multiple sub-regions that are respectively used for displaying the distribution statistic results of different types of physiological parameters.

There may be many historical values, and the numerical value of each historical value may be different. The historical values are displayed in a segmented mode, such that the medical personnel can ascertain the change of the historical values by means of comparing the distribution statistic results of different parameter-value partitions.

It should be noted that those information, such as the preset time duration corresponding to the historical value, the parameter-value partition to be displayed, and the physiological parameter to be displayed, may be either preset by the system or be independently selected and set by the user.

Specifically, in response to a selection instruction input by a user, the distribution statistic result of the historical values of the target physiological parameter selected by the user is displayed in the segment statistic region. The selection instruction represents the target physiological parameter that the user pays attention to, and the selection of the preset time duration corresponding to the historical values of the target physiological parameter.

A selection operation control can be provided on the monitoring interface for the user to input the selection instruction. For example, the selection operation control can provide options of the preset time duration in the form of a drop-down list, and the user can select a certain option. In response to the selection instruction input by the user, the time duration selected by the user is taken as the preset time duration. For another example, the selection operation control can provide options of multiple parameter-value partitions, the user can select a parameter-value partition, and the segment statistic region only displays the distribution statistic result corresponding to the selected parameter-value partition. As another example, the selection operation control may be multiple options of the physiological parameter, and in response to the selection instruction input by the user, the physiological parameter selected by the user is taken as the target physiological parameter.

The distribution statistic result may be displayed either in the form of text or in the form of a distribution statistic chart/table. The specific display mode is described as follows.

In some embodiments, after the distribution statistic result is obtained, a distribution statistic chart/table can be generated according to the parameter-value partition and the distribution statistic result.

The distribution statistic result is the number or the number ratio of the historical values, falling into the numerical value ranges, in the historical values of the physiological parameter within the preset time duration. A distribution statistic chart/table is generated based on the parameter-value partition and the distribution statistic result. For example, the distribution statistic chart/table is a histogram with the parameter-value partition and the distribution statistic result as two coordinate axes. The histogram may also be referred to as a statistic histogram. Alternatively, the distribution statistic chart/table may include a statistic chart such as a pie chart, or a statistic table.

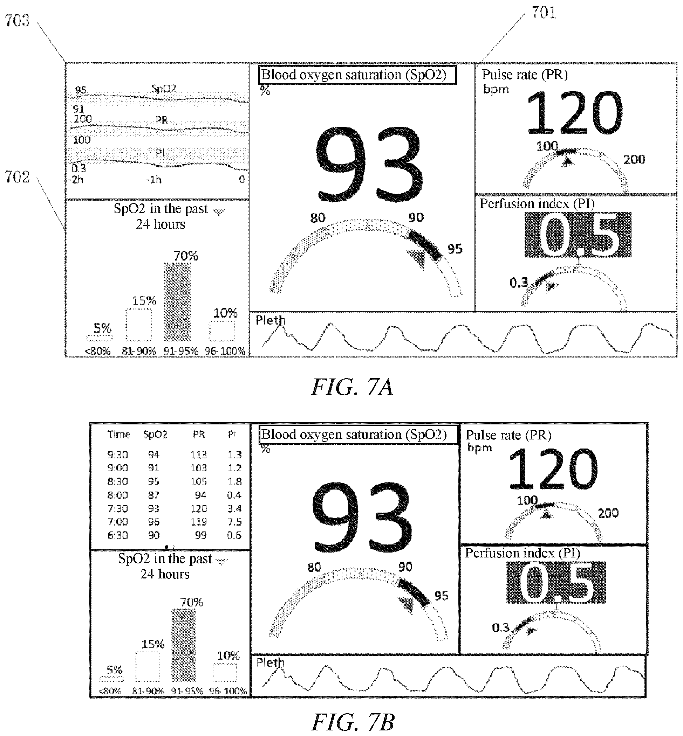

FIG. 7A shows an example of the monitoring interface. In addition to the state indication region 701, the monitoring interface further includes a segment statistic region 702. The segment statistic region 702 displays a histogram of the blood oxygen saturation. The histogram is obtained after the distribution statistics on the historical values of the blood oxygen saturation. It can be seen from the description of the histogram that the historical values are the parameter values within 24 hours (h) before the current time point. It should be noted that the time duration can be selected and set by the user, such as the downward triangle mark in FIG. 7A, and the user can select the time duration provided therefor by means of clicking the triangle mark.

The horizontal coordinates of the histogram are four parameter ranges of the blood oxygen saturation, which are respectively [0-80%], [81%-90%], [91%-95%] and [96%-100%]. The vertical coordinates of the histogram are the ratio of the historical values of the blood oxygen saturation in different parameter ranges of the blood oxygen saturation to the total historical values of the blood oxygen saturation. By means of viewing the histogram, the medical personnel can ascertain that the blood oxygen saturation statistic (SpO2 statistic) of the monitored object in the past 24 hours is as follows: the number with the blood oxygen saturation value less than 80% accounts for 5% of the total, the number with the blood oxygen saturation value greater than 81% and less than 90% accounts for 15% of the total, the number with the blood oxygen saturation value greater than 91% and less than 95% accounts for 70% of the total, and the number with the blood oxygen saturation value greater than 96% and less than 100% accounts for 10% of the total.

It should be noted that the distribution statistic result may correspond to multiple parameter-value partitions, and certain parameter-value partition(s) has special significance compared to other parameter-value partitions. The special meaning can be specifically that it can provide guidance for the medical personnel to determine the physiological state of the monitored object. For ease of description, the parameter-value partition with this special significance can be referred to as the target parameter-value partition. The target parameter-value partition may be a parameter-value partition corresponding to the normal situation, or a parameter-value partition corresponding to the abnormal situation, or a parameter-value partition desired to be reached after treatment by medical means (in which the parameter-value partition desired to be reached after treatment can be referred to as a treatment target partition, and the other parameter-value partitions can be referred to as non-treatment target partitions). Which parameter-value partition or parameter-value partitions the target parameter-value partition is may be preset by the system, or may be selected and set by the user.

In order to differentiate the target parameter-value partitions, a display pattern different from that for other parameter-value partitions is used to display the target parameter value partition and/or the distribution statistic result corresponding to target parameter value partition. Taking the blood oxygen saturation statistics shown in FIG. 7A as an example, if the treatment target partition is [91%-95%], the corresponding parameter range of [91%-95%] is filled with background color. Of course, the way to differentiate the displaying is not limited to color, and may also be performed by means of adding icons and symbols.

In order to provide the medical personnel more information to read, it is also possible to further display the representation graph, such as a trend chart and/or a trend table, of the historical values of the physiological parameter in addition to displaying the measured values of the physiological parameter.

Specifically, the trend chart and/or the trend table corresponding to the physiological parameter is obtained; and a trend display region is provided on the monitoring interface, and the trend chart and/or the trend table are displayed in the trend display region.

As shown in FIG. 7A, the monitoring interface further includes a trend display region 703 besides the state indication region 701 and the segment statistic region 702. The trend display region 703 contains trend charts of three physiological parameters, including the blood oxygen saturation (SpO2), the pulse rate (PR) and the perfusion index (PI). The trend chart may contain the normal parameter value range of the physiological parameter, and this range can be marked with shading and numerical values. As shown in FIG. 7A, it can be seen from the numerical scale that the normal parameter value range of the blood oxygen saturation (SpO2) includes 91%-95%, the normal parameter value range of the pulse rate (PR) includes 100 bpm to 200 bpm, and the normal parameter value range of the perfusion index (PI) is 1.0 or more.

The arrangement of the three trend charts is not limited thereto, and their order in the vertical direction can be arranged freely, or other arrangement directions can also be used.

In addition, the three trend charts are respectively generated from the historical values of three physiological parameters. It can be seen from -2h (hour) and -1h (hour) in FIG. 7A that the historical values are the parameter values within 2 hours before the current time point 0.

Alternatively, what is displayed in the trend display region is a trend table of the historical values of the physiological parameter. In the implementation of the trend table, the historical values of the physiological parameter may include the parameter values in a discontinuous time period. Specifically, the historical values include: the parameter values of the physiological parameter at multiple time points of different preset time durations from the current time point. It should be noted that the time points of the physiological parameter have different time durations from the current time point, but the time points of the physiological parameter may have the same time duration of interval therebetween. In brief, the parameter values of the physiological parameter are obtained at regular intervals within a historical time period.

FIG. 7B shows yet another example of the monitoring interface. The monitoring interface is different from FIG. 7A in that the trend display region displays the trend tables of the blood oxygen saturation (SpO2), the pulse rate (PR) and the perfusion index (PI). The trend table shows the parameter values of the blood oxygen saturation (SpO2), the pulse rate (PR) and the perfusion index (PI) at multiple different historical time points. The time points (Time) are respectively 6:30, 7:00, 7:30, 8:00, 8:30, 9:00, and 9:30, and the time duration of interval between the multiple historical time points is fixed, which can be 30 minutes. Of course, the time duration of interval can be other values, and is not limited thereto. It should be noted that the time points in FIG. 7B are arranged from top to bottom, and in turn are time points that are farther and farther from the current time point. Of course, the order of time points can be reversed, that is, the time points are getting closer and closer to the current time point from top to bottom.

With the change of time, the defined preset time duration can be changed, so the obtained historical values are changed, and the displayed distribution statistic result can refresh, where the refresh interval can be set by the user. Alternatively, after new measured value are obtained, the distribution statistic result synchronously refreshes, and the displayed chart/table refreshes.

The number of records displayed in the trend table can be either a preset fixed value or be set to the numerical value indicated by the instruction according to the user's setup instruction. If multiple statistic records cannot be displayed at the same time, they can be displayed on multiple screens, and the user can view other statistic records contained in other screens through touch operations.

It should be noted that the trend chart and the trend table are two different trend representations, and it is possible to preset which trend representation is fixedly displayed in the trend display region. Alternatively, the trend chart and the trend table can be switched mutually, and the triggering instruction of switching can be implemented by the user. Specifically, in response to an instruction input by the user, the trend chart and the trend table can be switched between each other in the trend display region.

In one example, a medical device may be provided with a button, such as a physical button or a virtual button displayed on the display screen, and the user can select whether to display the trend chart or the trend table by triggering the button.

In another example, when the display screen of the medical device is provided with the touch function, the way to input instructions can be a sliding operation on the display screen, such as sliding left or right, or sliding up or down. In order to facilitate the user to input accurate switching and sliding instructions, the trend display region may also contain a prompt icon to prompt the user which direction to slide to trigger the display of another trend representation.

The display screen of the medical device contains the monitoring interface. In the trend display region of the monitoring interface, a trend table is currently displayed, and the bottom of the trend table contains two circular icons. The circular icon on the left is darker, and the circular icon on the right is lighter, which indicates that the current display is the trend representation on the left, and sliding to the right is needed to trigger the display of another trend representation. Based on the prompt, the user triggers a sliding operation to the right on the display screen, and the content displayed in the trend display region can be switched from the trend table to a trend chart. At the same time, the circular icon changes, where the circular icon on the left is lighter, and the circular icon on the right is darker. Of course, the switching can be performed in an opposite direction to switch the trend chart to a trend table.

In addition, in order to give an alert for some abnormal parameter values in the physiological parameters in the trend table, an alarm can be given to the abnormal parameter values. Specifically, the parameter value of the physiological parameter in the trend table is monitored, and when the parameter value is determined to exceed the corresponding parameter threshold, the parameter value is displayed based on a preset display pattern; or the parameter value of the physiological parameter in the trend table is monitored, and if alarm information associated with the parameter value is monitored, the parameter value is displayed based on a preset display pattern; or the trend table is displayed in a display pattern that can differentiate the normal and abnormal parameter values of the physiological parameter in the trend table.

The alarm information may be an alarm condition defined according to an abnormal physiological state. If the parameter value of the physiological parameter triggers the alarm information, it is necessary to make a prompt for this parameter value. For example, the alarm information related to the blood oxygen saturation may include low blood oxygen saturation. If a certain parameter value of the blood oxygen saturation triggers a low blood oxygen saturation alarm, it is necessary to make a prompt for the parameter value of the blood oxygen saturation. For another example, the alarm information related to the pulse rate may include bradycardia, extreme bradycardia, or excessive slow heartbeat. If a certain parameter value of the pulse rate triggers any of the above alarm information, it is possible to make a prompt for the parameter value of the pulse rate.

In addition, in the trend chart, the normal numerical parameter value range corresponding to the physiological parameter may also be marked in a shading display mode; or in the trend chart, the normal numerical value range corresponding to the physiological parameter is marked in a ruler line mode.

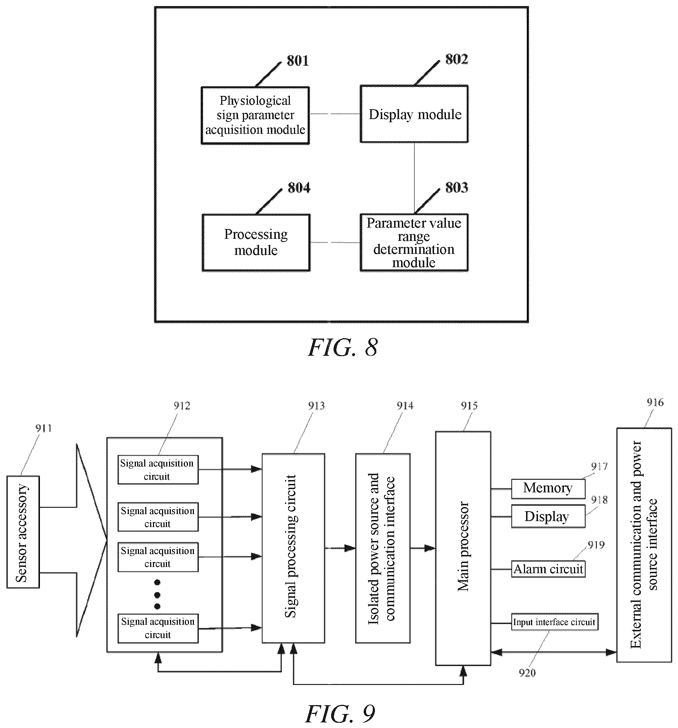

FIG. 8 shows a structure of an apparatus for displaying a physiological parameter provided in this disclosure. As shown in FIG. 8, the apparatus may specifically include: a physiological parameter acquisition module 801, a display module 802, a parameter-value range determination module 803, and a processing module 804.

The physiological parameter acquisition module 801 obtains a measured value of at least one physiological parameter of a monitored object, where the measured value of the physiological parameter is obtained from the monitored object by means of at least one sensor.

The display module 802 provides a monitoring interface, which includes a state indication region that displays at least one graphical state indicator including multiple indication blocks. The multiple indication blocks respectively correspond to multiple parameter value ranges of the physiological parameter, and the multiple indication blocks are displayed in the state indication region in an orderly arrangement according to the numerical value of the physiological parameter.

The parameter value range determination module 803 determines the parameter value range to which the measured value of the physiological parameter belongs.

The processing module 804 performs display and output operations with respect to an indication block of the graphical state indicator corresponding to the parameter value range to which the measured value belongs.

In an embodiment, the multiple indication blocks include a treatment target indication block corresponding to a parameter value range of the treatment target value of the physiological parameter.

In an embodiment, the apparatus for displaying the physiological parameter further includes a first jump module. The first jump module performs, when the measured value of the physiological parameter is determined to jump from a parameter value range to which the non-treatment target value belongs to a parameter value range to which the treatment target value belongs, display and output operations with respect to the treatment target indication block in a preset display pattern different from an original display pattern.

In an embodiment, the first jump module increases a display brightness of the treatment target indication block from an initial brightness value to a preset brightness value; and/or switches a display color of the treatment target indication block from an initial color to a preset color.

In an embodiment, the apparatus for displaying the physiological parameter further includes a second jump module. The second jump module cancels, when the measured value of the physiological parameter is determined to jump from a parameter value range to which the treatment-target value belongs to a parameter value range to which the non-treatment target value belongs, the preset display pattern and performs display and output operations with respect to the treatment target indication block in the original display pattern.

In an embodiment, the display module 802 displays the measured value in a state indication region of the monitoring interface; and/or displays a waveform chart and/or a trend chart corresponding to the physiological parameter in the state indication region of the monitoring interface.

In an embodiment, the display module 802 displays, if the measured value exceeds an alarm threshold of the physiological parameter, the measured value in the state indication region of the monitoring interface according to a preset alarm prompt mode.

In an embodiment, the display module 802 performs the display operations to differentiate the multiple indication blocks of the graphical state indicator in different display patterns.

In an embodiment, the display module 802 increases, in the graphical state indicator, the display brightness of the indication block corresponding to the parameter value range to which the measured value belongs from the initial brightness value to the preset brightness value; and/or switches the display color of the indication block corresponding to the parameter value range to which the measured value belongs from the initial color to the preset color.

In an embodiment, the multiple indication blocks include a target indication block, which represents the expected state of the monitored object with respect to the corresponding physiological parameter; and the display module 802 performs, if the parameter value range to which the measured value belongs is the parameter value range corresponding to the target indication block, display and output operations with respect to the target indication block in a display and output mode differentiated from other indication blocks.

In an embodiment, the display module 802 displays an indication icon at the position of the indication block corresponding to the parameter value range to which the measured value belongs.

In an embodiment, the graphical state indicator is in an arc shape or a straight-bar shape formed by connecting the multiple indication blocks.

In an embodiment, the display module 802 obtains historical values of at least one physiological parameter of the monitored object within a preset time duration; performs distribution statistics on the historical values based on at least one parameter-value partition; determines the distribution statistic result corresponding to the parameter-value partition; provides a segment statistic region on the monitoring interface, and displays the distribution statistic result in the segment statistic region.

In an embodiment, the distribution statistic result is that the number or the number ratio of the historical values of the physiological parameter falling into the parameter-value partition within the preset time duration.

In an embodiment, the display module 802 generates a distribution statistic chart/table based on the parameter-value partition and the distribution statistic result.

In an embodiment, the distribution statistic chart/table is a histogram with the parameter-value partition and the distribution statistic result as two coordinate axes.

In an embodiment, the apparatus for displaying the physiological parameter further includes a setup module. The setup module displays, in response to a selection instruction input by a user, the distribution statistic result of the historical values of the target physiological parameter selected by the user in the segment statistic region, where the selection instruction represents the target physiological parameter that the user pays attention to, and the selection of the preset time duration corresponding to the historical values thereof.

In an embodiment, the display module 802 determines the target parameter-value partition, and displays the target parameter-value partition and/or the distribution statistic result corresponding to the target parameter-value partition in a display pattern different from that for other parameter value partitions.

In an embodiment, the display module 802 obtains a trend chart and/or a trend table of the physiological parameter, and provides a trend display region on the monitoring interface to display the trend chart and/or the trend table in the trend display region.

In an embodiment, the display module 802 switches, in response to a switching instruction input by the user, between the trend chart and the trend table in the trend display region.

In an embodiment, the display module 802 monitors the parameter value of the physiological parameter in the trend table, and when the parameter value is determined to exceed the corresponding parameter threshold, displays the parameter value based on a preset display pattern. In an embodiment, the display module 802 monitors the parameter value of the physiological parameter in the trend table, and if alarm information associated with the parameter value is monitored, displays the parameter value based on a preset display pattern. In an embodiment, the display module 802 displays the trend table in a display pattern that can differentiate the normal and abnormal parameter values of the physiological parameter in the trend table. In an embodiment, in the trend chart, the display module 802 marks the normal numerical value range corresponding to the physiological parameter in a shading display mode; or in the trend chart, the display module 802 marks the normal numerical value range corresponding to the physiological parameter in a ruler line mode.

In an embodiment, the display module 802 displays the measured value in a measurement parameter display region on the monitoring interface, where the measurement parameter display region and the state indication region are two adjacent regions with a common boundary.

In an embodiment, the multiple indication blocks of the graphical state indicator are arranged in a straight-bar shape or a circular arc shape in an orderly manner; and the display module 802 displays a parameter pointer in the state indication region, with one end of the parameter pointer indicating the position of the indication block corresponding to the parameter value range to which the measured value belongs.

In an embodiment, the multiple indication blocks of the graphical state indicator are arranged in a circular arc shape in an orderly manner, and the central angle corresponding to the circular arc is less than 180 degrees.

In an embodiment, the multiple indication blocks of the graphical state indicator are arranged in a circular arc shape in an orderly manner, and the other end of the parameter pointer is displayed at the center position corresponding to the circular arc.

The present application further provides a monitoring system, which may specifically comprise: a processor and a display.

The display is configured to display information. The processor is configured to execute a program instruction to implement the steps in the method for displaying the physiological parameter in the forgoing embodiments.

The monitoring system mentioned in this disclosure may be either an independent device or a system formed by multiple independent devices interconnecting together. The monitoring system is not limited to monitors, but can also be invasive/non-invasive ventilators, anesthesia machines, defibrillators, nurse stations, central stations, and other devices with a monitoring function. Of course, it can also be a computer terminal or a mobile terminal that is equipped and executes clinical data monitoring and analysis software to implement the method provided in the above embodiment. The following embodiment mainly takes a monitor as an example for description.

A specific example of the monitor is shown in FIG. 9. FIG. 9 provides a system framework diagram of a parameter processing module in a multi-parameter monitor.

The multi-parameter monitor has an independent housing. A housing panel has a sensor interface zone in which multiple sensor interfaces are integrated for connecting with external physiological parameter sensor accessories 911. The housing panel further includes a small IXD display zone, a display 918, an input interface circuit 920, an alarm circuit 919 (such as an LED alarm zone), etc. The parameter processing module is used as an external communication and power source interface for communicating with a main unit and taking power from the main unit. The parameter processing module also supports a build-out parameter module, can form a plug-in monitor main unit by means of inserting the parameter module, can be used as part of the monitor, or can be connected to the main unit via a cable, with the build-out parameter module being used as an external accessory of the monitor. In addition, the multi-parameter monitor includes a memory 917 for storing computer programs and various data generated during the related monitoring process.

The internal circuit of the parameter processing module is disposed in the housing. As shown in FIG. 9 the internal circuit includes signal acquisition circuits 912 corresponding to at least two physiological parameters, a front-end signal processing circuit 913, and a main processor 915.

The main processor 915 can implement the steps related to processing in each apnea event monitoring method mentioned above.

The signal acquisition circuits 912 may be selected from an electrocardiogram circuit, a respiration circuit, a body temperature circuit, a blood oxygen saturation circuit, a non-invasive blood pressure circuit, an invasive blood pressure circuit, etc. The signal acquisition circuits 912 are respectively electrically connected to the corresponding sensor interfaces and are used to be electrically connected to the sensor accessories 911 corresponding to different physiological parameters, with an output end thereof being coupled to the front-end signal processor. A communication port of the front-end signal processor is coupled to the main processor, and the main processor is electrically connected to the external communication and power source interface.

Various physiological parameter measurement circuits can use common circuits in the prior art, the front-end signal processor completes sampling and analog-to-digital conversion of the output signal of the signal acquisition circuit, and outputs control signals to control the measurement process of the physiological signals. These parameters include but are not limited to parameters of electrocardiogram, respiration, body temperature, blood oxygen saturation, non-invasive blood pressure, and invasive blood pressure.

The front-end signal processor can be realized by a single-chip microcomputer or other semiconductor devices, and can also be realized by an ASIC or FPGA. The front-end signal processor can be powered by an isolated power source. The sampled data is simply processed and packaged, and then sent to the main processor through the isolated communication interface. For example, the front-end signal processor circuit can be coupled to the main processor 915 through the isolated power source and communication interface 914.

The reason that the front-end signal processor is powered by an isolated power source is that the DC/DC power source isolated by a transformer plays a role in isolating the patient from the power supply device, and the main purpose is: 1. isolating the patient, in which the application part is floated above the ground through the isolation transformer such that the leakage current of the patient is small enough; and 2. preventing the voltage or energy in the application of defibrillation or electric scalpel from affecting the boards and devices of the intermediate circuit such as the main control board (guaranteed by creepage distance and electrical clearance).

The main processor completes the calculation of physiological parameters, and sends the calculation results and waveforms of the parameters to the main unit (such as a main unit with a display, PC, central station, etc.) through the external communication and power source interface. The external communication and power source interface 916 may be one or a combination of local area network interfaces composed of Ethernet, Token Ring, Token Bus, and the optical fiber distributed data interface (FDDI) as the backbone of these three networks, may also be one or a combination of wireless interfaces such as infrared, Bluetooth, WIFI, and WMTS communication, or may also be one or a combination of wired data connection interfaces such as RS232 and USB.

The external communication and power source interface 916 may also be one or a combination of the wireless data transmission interface and the wired data transmission interface. The main unit may be any computer device such as the main unit of the monitor, an electrocardiograph, an ultrasonic diagnosis instrument, a computer, etc., and a monitoring device can be formed by means of installing with matching software. The main unit may also be a communication device, such as a mobile phone, and the parameter processing module sends data to a mobile phone that supports Bluetooth communication via a Bluetooth interface to realize remote data transmission.

In addition, the present application provides a readable storage medium with a computer program stored thereon, and when the computer program is executed by a processor, the above method for displaying various physiological parameters is implemented.

The description has been made with reference to various exemplary embodiments herein. However, those skilled in the art will recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

The terms "first", "second", etc. in the specification and the claims herein as well as the above accompanying drawings are used to distinguish different objects, rather than to describe a specific order. In addition, the terms "comprising", "having", and any variations thereof are intended to cover non-exclusive inclusion. For example, a process, a method, a system, a product, or a device that includes a series of steps or units is not limited to the listed steps or units, but optionally further includes unlisted steps or units, or optionally further includes other steps or units inherent in these processes, methods, or devices.

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions executed on a computer or other programmable data processing apparatus can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

The foregoing specific description has been described with reference to various embodiments. However, those skilled in the art will recognize that various modifications and changes can be made without departing from the scope of the present disclosure. Therefore, consideration of the present disclosure will be in an illustrative rather than a restrictive sense, and all such modifications will be included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "comprising", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communication connection, functional connection, and/or any other connection.

The above-mentioned examples merely represent several embodiments, giving specifics and details thereof, but should not be understood as limiting the scope of patent of this disclosure thereby. It should be noted that a person of ordinary skill in the art could also make several variations and improvements without departing from the concept of this disclosure, and these variations and improvements would all fall within the scope of protection of this disclosure. Therefore, the scope of protection of patent of this disclosure should be in accordance with the appended claims.

## Claims

1. A method for displaying a physiological parameter, comprising:
obtaining a measurement value of at least one physiological parameter of a monitored object, the measurement value of the physiological parameter being obtained from the monitored object by means of at least one sensor;
providing a monitoring interface, which comprises a state indication region that displays at least one graphical state indicator including multiple indication blocks, the multiple indication blocks respectively corresponding to multiple parameter value ranges of the physiological parameter, and the multiple indication blocks being displayed in the state indication region in an orderly arrangement according to a numerical value of the physiological parameter;
determining a parameter value range to which the measurement value of the physiological parameter belongs; and
performing display and output operations with respect to an indication block of the graphical state indicator corresponding to the parameter value range to which the measurement value belongs; and **characterized in that**,
the multiple indication blocks comprise a target indication block which represents an expected state of the monitored object with respect to a corresponding physiological parameter;
wherein the expected state is a treatment target state, and the treatment target state represents a state that the monitored object needs to reach after a treatment means in a monitoring scenario;
wherein, a parameter value range corresponding to the treatment target state is the parameter value range of a treatment target value, and the indication block corresponding to the parameter value range of the treatment target value is a treatment target indication block;
the method further comprises:
obtaining historical values of the at least one physiological parameter of the monitored object within a preset time duration;
performing distribution statistics on the historical values based on at least one parameter-value partition, and determining a distribution statistic result corresponding to the parameter-value partition, wherein the at least one parameter-value partition comprises a target parameter-value partition that corresponds to a parameter-value partition desired to be reached after treatment by medical means; and
providing a segment statistic region on the monitoring interface, and displaying the distribution statistic result in the segment statistic region.

2. The method of claim 1, **characterized in that** further comprising: when the measurement value of the physiological parameter is determined to change from a parameter value range to which a non-treatment target value belongs to the parameter value range to which the treatment target value belongs, performing display and output operations with respect to the treatment target indication block in a preset display pattern different from an original display pattern.

3. The method of claim 2, **characterized in that** performing display and output operations with respect to the treatment target indication block in a preset display pattern different from an original display pattern comprises: increasing a display brightness of the treatment target indication block from an initial brightness value to a preset brightness value; and/or switching a display color of the treatment target indication block from an initial color to a preset color.

4. The method of claim 2, **characterized in that** further comprising: when the measurement value of the physiological parameter is determined to change from the parameter value range to which the treatment target value belongs to the parameter value range to which the non-treatment target value belongs, canceling the preset display pattern, and performing display and output operations with respect to the treatment target indication block in the original display pattern.

5. The method of claim 1, **characterized in that** further comprising:
displaying the measurement value in the state indication region of the monitoring interface; and/or
displaying a trend chart and/or a waveform chart corresponding to the physiological parameter in the state indication region of the monitoring interface.

6. The method of claim 5, **characterized in that** displaying the measurement value in the state indication region of the monitoring interface comprises:
when the measurement value exceeds an alarm threshold of the physiological parameter, displaying the measurement value in the state indication region of the monitoring interface according to a preset alarm prompt mode.

7. The method of claim 1, **characterized in that** further comprising: differentiating the multiple indication blocks of the graphical state indicator by different display patterns.

8. The method of claim 1, **characterized in that** further comprising: in the graphical state indicator, increasing a display brightness of the indication block corresponding to the parameter value range to which the measurement value belongs from an initial brightness value to a preset brightness value; and/or switching a display color of the indication block corresponding to the parameter value range to which the measurement value belongs from an initial color to a preset color.

9. The method of claim 1, **characterized in that** performing display and output operations with respect to an indication block of the graphical state indicator corresponding to the parameter value range to which the measurement value belongs comprises:
when the parameter value range to which the measurement value belongs is the parameter value range corresponding to the treatment target indication block, performing display and output operations with respect to the treatment target indication block in a display and output mode different from other indication blocks.

10. The method of claim 1, **characterized in that** performing display and output operations with respect to an indication block of the graphical state indicator corresponding to the parameter value range to which the measurement value belongs comprises:
displaying an indication icon at a position of the indication block corresponding to the parameter value range to which the measurement value belongs.

11. The method of claim 1, **characterized in that** further comprising: in response to a selection instruction input by a user, displaying the distribution statistic result of the historical values of a target physiological parameter selected by the user in the segment statistic region, the selection instruction representing the target physiological parameter that the user pays attention to, and a selection of the preset time duration corresponding to the historical values of the target physiological parameter.

12. The method of claim 1, **characterized in that** further comprising: determining a target parameter-value partition, and displaying the target parameter-value partition and/or the distribution statistic result corresponding to the target parameter-value partition in a display pattern different from that for other parameter-value partitions.

13. The method of claim 1, **characterized in that** further comprising: displaying the measurement value in a measurement parameter display region on the monitoring interface, the measurement parameter display region and the state indication region being two adjacent regions with a common boundary;
preferably the multiple indication blocks of the graphical state indicator are arranged in a straight-bar shape or a circular arc shape in an orderly manner; and the method further comprises:
displaying a parameter pointer in the state indication region, with one end of the parameter pointer indicating a position of the indication block corresponding to the parameter value range to which the measurement value belongs; preferably when the multiple indication blocks of the graphical state indicator are arranged in a circular arc shape in an orderly manner, a central angle corresponding to the circular arc is less than 180 degrees and the other end of the parameter pointer is displayed at a center position corresponding to the circular arc.

14. The method of claim 1, **characterized in that**, further comprising adding a prompt in the graphical state indicator at a corresponding position of the treatment target indication block; preferably, the prompt is in text form, a picture or a symbol.

## Patentansprüche

1. Ein Verfahren zur Anzeige eines physiologischen Parameters, das Folgendes umfasst:
das Ermitteln eines Messwerts mindestens eines physiologischen Parameters eines überwachten Patienten, wobei der Messwert des physiologischen Parameters von dem überwachten Patienten mittels mindestens eines Sensors ermittelt wird;
das Bereitstellen einer Überwachungsschnittstelle, die einen Zustandsanzeigebereich umfasst, der mindestens einen grafischen Zustandsindikator mit mehreren Anzeigeblöcken anzeigt, wobei die mehreren Anzeigeblöcke jeweils mehreren Parameterwertbereichen des physiologischen Parameters entsprechen und die mehreren Anzeigeblöcke im Zustandsanzeigebereich in einer geordneten Anordnung entsprechend einem numerischen Wert des physiologischen Parameters angezeigt werden;
das Ermitteln eines Parameterwertbereichs, zu dem der Messwert des physiologischen Parameters gehört; und
das Durchführen von Anzeige- und Ausgabevorgängen in Bezug auf einen Anzeigeblock des grafischen Zustandsindikators, der dem Parameterwertbereich entspricht, zu dem der Messwert gehört; **dadurch gekennzeichnet, dass**,
die mehreren Anzeigeblöcke einen Zielanzeigeblock umfassen, der einen erwarteten Zustand des überwachten Patienten in Bezug auf einen entsprechenden physiologischen Parameter darstellt;
wobei der erwartete Zustand ein Behandlungszielzustand ist und der Behandlungszielzustand einen Zustand darstellt, den der überwachte Patient nach einer Behandlungsmaßnahme in einem Überwachungsszenario erreichen muss;
wobei ein dem Behandlungszielzustand entsprechender Parameterwertbereich der Parameterwertbereich eines Behandlungszielwerts ist und der dem Parameterwertbereich des Behandlungszielwerts entsprechende Anzeigeblock ein Behandlungszielanzeigeblock ist;
das Verfahren ferner Folgendes umfasst:
das Ermitteln von historischen Werten des mindestens einen physiologischen Parameters des überwachten Patienten innerhalb einer voreingestellten Zeitdauer;
das Erstellen von Verteilungsstatistiken über die historischen Werte auf der Grundlage mindestens einer Parameterwertpartition und das Ermitteln eines der Parameterwertpartition entsprechenden Verteilungsstatistikergebnisses, wobei die mindestens eine Parameterwertpartition eine Zielparameterwertpartition umfasst, die einer Parameterwertpartition entspricht, die nach einer Behandlung durch medizinische Mittel erreicht werden soll; und
das Bereitstellen eines Segmentstatistikbereichs auf der Überwachungsschnittstelle und das Anzeigen des Verteilungsstatistikergebnisses in dem Segmentstatistikbereich.

2. Das Verfahren nach Anspruch 1, **dadurch gekennzeichnet, dass** es ferner Folgendes umfasst: wenn festgestellt wird, dass sich der Messwert des physiologischen Parameters von einem Parameterwertbereich, zu dem ein Nicht-Behandlungszielwert gehört, zu dem Parameterwertbereich ändert, zu dem der Behandlungszielwert gehört, das Durchführen von Anzeige- und Ausgabevorgängen in Bezug auf den Behandlungszielanzeigeblock in einem voreingestellten Anzeigemuster, das sich von einem ursprünglichen Anzeigemuster unterscheidet.

3. Das Verfahren nach Anspruch 2, **dadurch gekennzeichnet, dass** das Durchführen von Anzeige- und Ausgabevorgängen in Bezug auf den Behandlungszielanzeigeblock in einem voreingestellten Anzeigemuster, das sich von einem ursprünglichen Anzeigemuster unterscheidet, Folgendes umfasst: das Erhöhen einer Anzeigehelligkeit des Behandlungszielanzeigeblocks von einem Anfangshelligkeitswert auf einen voreingestellten Helligkeitswert; und/oder das Umschalten einer Anzeigefarbe des Behandlungszielanzeigeblocks von einer Anfangsfarbe auf eine voreingestellte Farbe.

4. Das Verfahren nach Anspruch 2, **dadurch gekennzeichnet, dass** es ferner Folgendes umfasst: wenn festgestellt wird, dass sich der Messwert des physiologischen Parameters von dem Parameterwertbereich, zu dem der Behandlungszielwert gehört, zu dem Parameterwertbereich ändert, zu dem der Nicht-Behandlungszielwert gehört, das Aufheben des voreingestellten Anzeigemusters und das Durchführen von Anzeige- und Ausgabevorgängen in Bezug auf den Behandlungsziel-Anzeigeblock in dem ursprünglichen Anzeigemuster.

5. Das Verfahren nach Anspruch 1, **dadurch gekennzeichnet, dass** es ferner Folgendes umfasst:
das Anzeigen des Messwerts im Statusanzeigebereich der Überwachungsschnittstelle; und/oder
das Anzeigen eines Trenddiagramms und/oder eines Wellenformdiagramms, das dem physiologischen Parameter entspricht, im Statusanzeigebereich der Überwachungsschnittstelle.

6. Das Verfahren nach Anspruch 5, **dadurch gekennzeichnet, dass** das Anzeigen des Messwerts im Statusanzeigebereich der Überwachungsschnittstelle Folgendes umfasst:
wenn der Messwert einen Alarmschwellenwert des physiologischen Parameters überschreitet, das Anzeigen des Messwerts im Statusanzeigebereich der Überwachungsschnittstelle gemäß einem voreingestellten Alarmwarnmodus.

7. Das Verfahren nach Anspruch 1, **dadurch gekennzeichnet, dass** es ferner Folgendes umfasst: das Unterscheiden der mehreren Anzeigeblöcke der grafischen Zustandsanzeige durch unterschiedliche Anzeigemuster.

8. Das Verfahren nach Anspruch 1, **dadurch gekennzeichnet, dass** es ferner Folgendes umfasst: in der grafischen Zustandsanzeige das Erhöhen einer Anzeigeleuchtkraft des Anzeigeblocks, der dem Parameterwertbereich entspricht, zu dem der Messwert gehört, von einem Anfangswert auf einen voreingestellten Wert; und/oder das Umschalten einer Anzeigefarbe des Anzeigeblocks, der dem Parameterwertbereich entspricht, zu dem der Messwert gehört, von einer Anfangsfarbe auf eine voreingestellte Farbe.

9. Das Verfahren nach Anspruch 1, **dadurch gekennzeichnet, dass** das Durchführen von Anzeige- und Ausgabevorgängen in Bezug auf einen Anzeigeblock der grafischen Zustandsanzeige, der dem Parameterwertbereich entspricht, zu dem der Messwert gehört, Folgendes umfasst:
wenn der Parameterwertbereich, zu dem der Messwert gehört, der dem Behandlungszielanzeigeblock entsprechende Parameterwertbereich ist, werden Anzeige- und Ausgabevorgänge in Bezug auf den Behandlungszielanzeigeblock in einem Anzeige- und Ausgabemodus ausgeführt, der sich von dem anderer Anzeigeblöcke unterscheidet.

10. Das Verfahren nach Anspruch 1, **dadurch gekennzeichnet, dass** das Durchführen von Anzeige- und Ausgabevorgängen in Bezug auf einen Anzeigeblock der grafischen Zustandsanzeige, der dem Parameterwertbereich entspricht, zu dem der Messwert gehört, Folgendes umfasst:
das Anzeigen eines Anzeigesymbols an einer Stelle des Anzeigeblocks, die dem Parameterwertbereich entspricht, zu dem der Messwert gehört.

11. Das Verfahren nach Anspruch 1, **dadurch gekennzeichnet, dass** es ferner Folgendes umfasst: als Reaktion auf eine von einem Benutzer eingegebene Auswahlanweisung das Anzeigen des Verteilungsstatistikergebnisses der historischen Werte eines vom Benutzer ausgewählten physiologischen Zielparameters im Segmentstatistikbereich, wobei die Auswahlanweisung den physiologischen Zielparameter darstellt, auf den der Benutzer sein Augenmerk richtet, sowie eine Auswahl der voreingestellten Zeitdauer, die den historischen Werten des physiologischen Zielparameters entspricht.

12. Das Verfahren nach Anspruch 1, **dadurch gekennzeichnet, dass** es ferner Folgendes umfasst: das Ermitteln einer Zielparameterwertpartition und Anzeigen der Zielparameterwertpartition und/oder des der Zielparameterwertpartition entsprechenden Verteilungsstatistikergebnisses in einem Anzeigemuster, das sich von dem für andere Parameterwertpartitionen unterscheidet.

13. Das Verfahren nach Anspruch 1, **dadurch gekennzeichnet, dass** es ferner Folgendes umfasst: das Anzeigen des Messwerts in einem Messparameteranzeigebereich auf der Überwachungsschnittstelle, wobei der Messparameteranzeigebereich und der Zustandsanzeigebereich zwei benachbarte Bereiche mit einer gemeinsamen Grenze sind;
vorzugsweise die mehreren Anzeigeblöcke der grafischen Zustandsanzeige in einer geradlinigen oder bogenförmigen Anordnung angeordnet sind; und das Verfahren ferner Folgendes umfasst:
das Anzeigen eines Parameterzeigers im Zustandsanzeigebereich, wobei ein Ende des Parameterzeigers eine Position des Anzeigeblocks anzeigt, die dem Parameterwertbereich entspricht, zu dem der Messwert gehört; vorzugsweise, wenn die mehreren Anzeigeblöcke der grafischen Zustandsanzeige in einer geordneten Weise in Form eines Kreisbogens angeordnet sind, beträgt der dem Kreisbogen entsprechende Zentriwinkel weniger als 180 Grad, und wird das andere Ende des Parameterzeigers an einer dem Kreisbogen entsprechenden Mittelposition angezeigt.

14. Das Verfahren nach Anspruch 1, **dadurch gekennzeichnet, dass** es ferner das Hinzufügen einer Eingabeaufforderung in der grafischen Zustandsanzeige an einer entsprechenden Position des Behandlungszielanzeigeblocks umfasst; vorzugsweise liegt die Eingabeaufforderung in Textform, als Bild oder als Symbol vor.

## Revendications

1. Procédé pour l'affichage d'un paramètre physiologique, comprenant les étapes consistant à :
obtenir une valeur de mesure d'au moins un paramètre physiologique d'un objet surveillé, la valeur de mesure du paramètre physiologique étant obtenue à partir de l'objet surveillé au moyen d'au moins un capteur ;
fournir une interface de surveillance, laquelle comprend une zone d'indication d'état qui affiche au moins un indicateur d'état graphique incluant de multiples blocs d'indication, les multiples blocs d'indication correspondant respectivement à de multiples plages de valeur de paramètre du paramètre physiologique, et les multiples blocs d'indication étant affichés dans la zone d'indication d'état suivant une disposition ordonnée selon une valeur numérique du paramètre physiologique ;
déterminer une plage de valeur de paramètre à laquelle la valeur de mesure du paramètre physiologique appartient ; et
réaliser des opérations de sortie et d'affichage par rapport à un bloc d'indication de l'indicateur d'état graphique correspondant à la plage de valeur de paramètre à laquelle la valeur de mesure appartient ; et **caractérisé en ce que**,
les multiples blocs d'indication comprennent un bloc d'indication cible lequel représente un état souhaité de l'objet surveillé par rapport à un paramètre physiologique correspondant ;
où l'état souhaité est un état cible de traitement, et l'état cible de traitement représente un état que l'objet surveillé doit atteindre après un moyen de traitement dans un scénario de surveillance ;
où, une plage de valeur de paramètre correspondant à l'état cible de traitement est la plage de valeur de paramètre d'une valeur cible de traitement, et le bloc d'indication correspondant à la plage de valeur de paramètre de la valeur cible de traitement est un bloc d'indication cible de traitement ;
le procédé comprend en outre :
le fait d'obtenir des valeurs antérieures du au moins un paramètre physiologique de l'objet surveillé sur une période prédéfinie ;
le fait de réaliser des statistiques de distribution sur les valeurs antérieures sur la base d'au moins une répartition de valeur de paramètre, et de déterminer un résultat de statistique de distribution correspondant à la répartition de valeur de paramètre, où la au moins une répartition de valeur de paramètre comprend une répartition de valeur de paramètre cible qui correspond à une répartition de valeur de paramètre qu'il est souhaité d'atteindre après traitement par un moyen médical ; et
le fait de fournir une zone de statistique de segment sur l'interface de surveillance, et d'afficher le résultat de statistique de distribution dans la zone de statistique de segment.

2. Procédé selon la revendication 1, **caractérisé en ce qu'**il comprend en outre : lorsqu'il est déterminé que la valeur de mesure du paramètre physiologique passe d'une plage de valeur de paramètre à laquelle une valeur non cible de traitement appartient à la plage de valeur de paramètre à laquelle la valeur cible de traitement appartient, le fait de réaliser des opérations de sortie et d'affichage par rapport au bloc d'indication cible de traitement selon un motif d'affichage prédéfini différent d'un motif d'affichage d'origine.

3. Procédé selon la revendication 2, **caractérisé en ce que** le fait de réaliser des opérations de sortie et d'affichage par rapport au bloc d'indication cible de traitement selon un motif d'affichage prédéfini différent d'un motif d'affichage d'origine comprend : le fait d'augmenter une luminosité d'affichage du bloc d'indication cible de traitement d'une valeur de luminosité initiale à une valeur de luminosité prédéfinie ; et/ou de changer une couleur d'affichage du bloc d'indication cible de traitement d'une couleur initiale à une couleur prédéfinie.

4. Procédé selon la revendication 2, **caractérisé en ce qu'**il comprend en outre : lorsqu'il est déterminé que la valeur de mesure du paramètre physiologique passe de la plage de valeur de paramètre à laquelle la valeur cible de traitement appartient à la plage de valeur de paramètre à laquelle la valeur non cible de traitement appartient, le fait d'effacer le motif d'affichage prédéfini, et de réaliser des opérations de sortie et d'affichage par rapport au bloc d'indication cible de traitement selon le motif d'affichage d'origine.

5. Procédé selon la revendication 1, **caractérisé en ce qu'**il comprend en outre :
le fait d'afficher la valeur de mesure dans la zone d'indication d'état de l'interface de surveillance ; et/ou
le fait d'afficher un graphique de tendance et/ou un graphique de forme d'onde correspondant au paramètre physiologique dans la zone d'indication d'état de l'interface de surveillance.

6. Procédé selon la revendication 5, **caractérisé en ce que** le fait d'afficher la valeur de mesure dans la zone d'indication d'état de l'interface de surveillance comprend :
lorsque la valeur de mesure dépasse un seuil d'alarme du paramètre physiologique, le fait d'afficher la valeur de mesure dans la zone d'indication d'état de l'interface de surveillance selon un mode d'alerte d'alarme prédéfini.

7. Procédé selon la revendication 1, **caractérisé en ce qu'**il comprend en outre :
le fait de différencier les multiples blocs d'indication de l'indicateur d'état graphique par différents motifs d'affichage.

8. Procédé selon la revendication 1, **caractérisé en ce qu'**il comprend en outre : dans l'indicateur d'état graphique, le fait d'augmenter une luminosité d'affichage du bloc d'indication correspondant à la plage de valeur de paramètre à laquelle la valeur de mesure appartient d'une valeur de luminosité initiale à une valeur de luminosité prédéfinie ; et/ou de changer une couleur d'affichage du bloc d'indication correspondant à la plage de valeur de paramètre à laquelle la valeur de mesure appartient d'une couleur initiale à une couleur prédéfinie.

9. Procédé selon la revendication 1, **caractérisé en ce que** le fait de réaliser des opérations de sortie et d'affichage par rapport à un bloc d'indication de l'indicateur d'état graphique correspondant à la plage de valeur de paramètre à laquelle la valeur de mesure appartient comprend :
lorsque la plage de valeur de paramètre à laquelle la valeur de mesure appartient est la plage de valeur de paramètre correspondant au bloc d'indication cible de traitement, le fait de réaliser des opérations de sortie et d'affichage par rapport au bloc d'indication cible de traitement dans un mode de sortie et d'affichage différent des autres blocs d'indication.

10. Procédé selon la revendication 1, **caractérisé en ce que** le fait de réaliser des opérations de sortie et d'affichage par rapport à un bloc d'indication de l'indicateur d'état graphique correspondant à la plage de valeur de paramètre à laquelle la valeur de mesure appartient comprend :
le fait d'afficher une icône d'indication à une position du bloc d'indication correspondant à la plage de valeur de paramètre à laquelle la valeur de mesure appartient.

11. Procédé selon la revendication 1, **caractérisé en ce qu'**il comprend en outre : en réponse à une instruction de sélection entrée par un utilisateur, le fait d'afficher le résultat de statistique de distribution des valeurs antérieures d'un paramètre physiologique cible sélectionné par l'utilisateur dans la zone de statistique de segment, l'instruction de sélection représentant le paramètre physiologique cible auquel l'utilisateur prête attention, et une sélection de la période prédéfinie correspondant aux valeurs antérieures du paramètre physiologique cible.

12. Procédé selon la revendication 1, **caractérisé en ce qu'**il comprend en outre :
le fait de déterminer une répartition de valeur de paramètre cible, et le fait d'afficher la répartition de valeur de paramètre cible et/ou le résultat de statistique de distribution correspondant à la répartition de valeur de paramètre cible selon un motif d'affichage différent de celui d'autres répartitions de valeur de paramètre.

13. Procédé selon la revendication 1, **caractérisé en ce qu'**il comprend en outre :
le fait d'afficher la valeur de mesure dans une zone d'affichage de paramètre de mesure sur l'interface de surveillance, la zone d'affichage de paramètre de mesure et la zone d'indication d'état étant deux zones adjacentes partageant une limite commune ;
de préférence les multiples blocs d'indication de l'indicateur d'état graphique sont disposés selon une forme de barre droite ou une forme d'arc circulaire d'une manière ordonnée ; et le procédé comprend en outre :
le fait d'afficher un pointeur de paramètre dans la zone d'indication d'état, une première extrémité du pointeur de paramètre indiquant une position du bloc d'indication correspondant à la plage de valeur de paramètre à laquelle la valeur de mesure appartient ; de préférence lorsque de multiples blocs d'indication de l'indicateur d'état graphique sont agencés selon une forme d'arc circulaire d'une manière ordonnée, un angle central correspondant à l'arc circulaire est inférieur à 180 degrés et l'autre extrémité du pointeur de paramètre est affichée à une position de centre correspondant à l'arc circulaire.

14. Procédé selon la revendication 1, **caractérisé en ce qu'**il comprend en outre le fait d'ajouter une alerte dans l'indicateur d'état graphique à une position correspondante du bloc d'indication cible de traitement ; de préférence, l'alerte se présente sous la forme d'un texte, d'une image ou d'un symbole.
